# EUROPÄISCHE PATENTSCHRIFT

(11) **EP 1 693 415 B1**
(45) Veröffentlichungstag und Bekanntmachung des Hinweises auf die Patenterteilung: **27.02.2019**
(21) Anmeldenummer: 05112990.6
(22) Anmeldetag: 27.12.2005
(51) Int. Cl.: C08L 53/00, C09D 153/00, B32B 27/34, C08G 69/14

(54) **POLYMERPULVER MIT BLOCKPOLYETHERAMID UND DIE VERWENDUNG IN EINEM FORMGEBENDEN VERFAHREN UND FORMKÖRPER HERGESTELLT AUS DIESEM POLYMERPULVER**
POLYMER POWDER WITH BLOCKPOLYETHERAMIDE, AND THE USE IN A PROCESS AND MOULDING PRODUCED FROM THIS POLYMER POWDER
POUDRE DE POLYMÈRE AVEC COPOLYETHERAMIDE À BLOCS, ET SON UTILISATION DANS UN PROCÉDÉ ET PRODUIT DE MOULAGE.

(30) Priorität: 19.02.2005 DE 102005008044
(43) Veröffentlichungstag der Anmeldung: 23.08.2006
(60) Teilanmeldung: 07113476.1
(73) Patentinhaber: Evonik Degussa GmbH, 45128 Essen (DE)
(72) Erfinder: Monsheimer, Sylvia, 45721, Haltern am See (DE); Baumann, Frank-Erich, 48249, Dülmen (DE); Grebe, Maik, 44805, Bochum (DE); Simon, Ulrich, 44625, Herne (DE); Hessel, Sigrid, 45721, Haltern am See (DE)

(56) Entgegenhaltungen:
- EP-A- 0 290 761
- EP-A- 0 577 348
- EP-A- 0 610 122
- EP-A- 0 911 142
- WO-A-2005/109994
- DE-A1- 4 433 118
- US-A- 6 110 411
- US-A1- 2005 014 842

## Beschreibung

Die zügige Bereitstellung von Prototypen ist eine in der jüngsten Zeit häufig gestellte Aufgabe. Besonders geeignet sind Verfahren, die auf der Basis von pulverförmigen Werkstoffen arbeiten, und bei denen schichtweise durch selektives Aufschmelzen und Verfestigen die gewünschten Strukturen hergestellt werden. Auf Stützkonstruktionen bei Überhängen und Hinterschnitten kann dabei verzichtet werden, da das die aufgeschmolzenen Bereiche umgebende Pulverbett ausreichende Stützwirkung bietet. Ebenso entfällt die Nacharbeit, Stützen zu entfernen. Die Verfahren sind auch für die Herstellung von Kleinserien geeignet.

Die Erfindung betrifft ein Polymerpulver mit Blockpolyetheramid auf Basis einer Oligoamiddicarbonsäure und Polyetheraminen, bevorzugt auf Basis einer Oligoamiddicarbonsäure und Polyetherdiaminen, die Verwendung dieses Pulvers in formgebenden Verfahren, sowie Formkörper, hergestellt durch ein schichtweise arbeitendes Verfahren, mit welchem selektiv Bereiche einer Pulverschicht aufgeschmolzen werden, unter Verwendung dieses Pulvers. Nach Abkühlen und Verfestigen der zuvor Schicht für Schicht aufgeschmolzenen Bereiche kann der Formkörper dem Pulverbett entnommen werden.

Die Selektivität der schichtweise arbeitenden Verfahren kann dabei beispielsweise über den Auftrag von Suszeptoren, Absorbern, Inhibitoren oder durch Masken oder über fokussierte Energieeinbringung, wie beispielsweise durch einen Laserstrahl, oder über Glasfasern erfolgen. Der Energieeintrag wird über elektromagnetische Strahlung erreicht.

Im Folgenden werden einige Verfahren beschrieben, mit denen aus dem erfindungsgemäßen Pulver erfindungsgemäße Formteile hergestellt werden können, ohne dass die Erfindung darauf beschränkt werden soll.

Ein Verfahren, welches besonders gut für den Zweck des Rapid Prototypings geeignet ist, ist das selektive Laser-Sintern. Bei diesem Verfahren werden Kunststoffpulver in einer Kammer selektiv kurz mit einem Laserstrahl belichtet, wodurch die Pulver-Partikel, die von dem Laserstrahl getroffen werden, schmelzen. Die geschmolzenen Partikel laufen ineinander und erstarren schnell wieder zu einer festen Masse. Durch wiederholtes Belichten von immer neu aufgebrachten Schichten können mit diesem Verfahren dreidimensionale Körper einfach und schnell hergestellt werden.

Das Verfahren des Laser-Sinterns (Rapid Prototyping) zur Darstellung von Formkörpern aus pulverförmigen Polymeren wird ausführlich in der Patentschriften US 6 136 948 und WO 96/06881 (beide DTM Corporation) beschrieben. Eine Vielzahl von Polymeren und Copolymeren wird für diese Anwendung beansprucht, wie z. B. Polyacetat, Polypropylen, Polyethylen, lonomere und Polyamid.

Andere gut geeignete Verfahren sind das SIV-Verfahren, wie in WO 01/38061 beschrieben, oder ein Verfahren wie in EP 1 015 214 beschrieben. Beide Verfahren arbeiten mit einer flächigen Infrarotheizung zum Aufschmelzen des Pulvers. Die Selektivität des Aufschmelzens wird bei ersterem durch die Auftragung eines Inhibitors, beim zweiten Verfahren durch eine Maske erreicht. Ein weiteres Verfahren ist in DE 103 11 438 beschrieben. Bei diesem wird die zum Verschmelzen benötigte Energie durch einen Mikrowellengenerator eingebracht, und die Selektivität wird durch Auftragen eines Suszeptors erreicht.

Weitere geeignete Verfahren sind solche, die mit einem Absorber arbeiten, der entweder im Pulver enthalten ist, oder der per Inkjet-Verfahren aufgetragen wird wie in DE 10 2004 012 682.8, DE 10 2004 012 683.6 und DE 10 2004 020 452.7 beschrieben.

Für die genannten Rapid-Prototyping- bzw. Rapid-Manufacturing-Verfahren (RP- oder RM-Verfahren) können pulverförmige Substrate, insbesondere Polymere, vorzugsweise ausgewählt aus Polyester, Polyvinylchlorid, Polyacetal, Polypropylen, Polyethylen, Polystyrol, Polycarbonat, Poly-(N-methylmethacrylimide) (PMMI), Polymethylmethacrylat (PMMA), lonomer, Polyamid, oder Gemische davon eingesetzt werden.

In US 6,110,411 werden speziell für das Lasersintern Pulver von Blockcopolymeren beschrieben, die aus einem Hart- und einem Weichsegment bestehen, wobei der Hartblock einen Polyamidbaustein enthalten kann, der Weichblock jedoch aus einer anderen Komponente besteht, nämlich aus Ether- und Estereinheiten. Die Struktur der Weichsegmente wird allgemein durch die Formeln (1) oder (2) beschrieben:

(1) -O-G-O-C(O)-R-C(O)-

(2) -O-D-O-C(O)-R-C(O)-

in denen R für den Rest einer Dicarbonsäure und G bzw. D für den Rest eines Glycols bzw. langekettigen Diols/polyetherdiols, der nach Abstraktion der terminalen Hydroxylgruppen verbleibt. Die in der gleichen Druckschrift erwähnte Eignung von Polyether-block-amiden der PEBAX® - Reihe bezieht sich ebenfalls auf Polyamid-Elastomere, in denen Polyether- und aliphatische Polyamid-Segmente durch Estergruppen miteinander verknüpft sind.

Außerdem müssen die dort beschriebenen Pulver ein Rieselhilfsmittel aufweisen und eine Glastemperatur kleiner als 50 °C besitzen. Stabile Blockcopolymere mit definierter Struktur können jedoch auf Basis von Polyamiden nicht hergestellt werden, mit Ausnahme der unter die zitierte Anmeldung fallenden Polyetheresteramiden (PEBA) und der nicht unter die zitierte Anmeldung fallenden Polyetheraminen (PEA). Üblicherweise finden in der Polyamide enthaltenden Schmelze Umamidierungsreaktionen statt, bis sich wieder eine statistische Verteilung der Monomeren eingestellt hat.

In DE 44 33 118 werden Polymerblends betrachtet. Ein Blend ist aber eine unter definierten Temperatur- und Scherbedingungen aus zwei oder mehr Polymeren in der Schmelze hergestellte Mischung, die üblicherweise zu Granulaten verarbeitet wird. Die einzelnen Polymerketten werden dabei untereinander vermischt ("intermolekular"), innerhalb einer Kette findet jedoch keine Kombination der Ausgangskomponenten statt (Definition s. beispielsweise Sächtling Kunstofftaschenbuch, 24, Auflage, S, 7 ff.).

In EP 0 060 579 A1 wird ein Polyetheramin in Verbindung mit einem Polyamid 6 oder 66 beschrieben. Die Lösungsviskosität der betrachteten Copolymere liegt zwischen 2 und 3,5. Das Material ist wegen der erhöhten Wasseraufnahme nicht für die oben beschriebenen werkzeuglosen Herstellprozesse geeignet und lässt sich nicht oder nur schwer vermahlen.

In US 5,296,062 werden Pulver mit deutlich verschiedenen Schmelzpunkten behandelt. Hauptanwendung ist das Verkleben einer höherschmelzenden Metallkomponente mit einer niedriger schmelzenden Metall- oder Kunststoffkomponente. Dabei können die Partikel nebeneinander vorliegen, oder das niedriger schmelzende wird als Coating auf die andere Komponente aufgebracht. Es handelt sich dabei nicht um eine homogene Mischung innerhalb eines Pulverpartikels. In US 6,143,852 wird ein Copolymeres aus Methylmethacrylat mit C2-C10-Alkylmethacrylat beschrieben, welches man durch Dispersionspolymerisation erhält. Dadurch erhält man sehr kleine Partikel und eine sehr enge Korngrößenverteilung. Kleine Partikel sind aber wegen ihrer schlechten Fließfähigkeit für das Lasersintern weniger gut geeignet; eine enge Kornverteilung wie beschrieben führt zur erschwerten Verarbeitung in einem schichtweise arbeitenden Verfahren, bei dem selektiv Bereiche aufgeschmolzen werden, und zwar durch ein kleines Verarbeitungsfenster, welches im Extremfall zur Nichteignung führen kann.

In WO 95/11006 wird ein für das Lasersintern geeignetes Polymerpulver beschrieben, das bei der Bestimmung des Schmelzverhaltens durch Differential Scanning Calorimetry bei einer Scanning rate von 10 bis 20 °C/min keine Überlappung des Schmelze- und Rekristallisationspeaks zeigt, einen ebenfalls durch DSC bestimmten Kristallinitätsgrad von 10 bis 90 % aufweist, ein zahlenmäßiges Mittel des Molekulargewichts Mn von 30 000 bis 500 000 hat und dessen Quotient Mw/Mn im Bereich von 1 bis 5 liegt.

In EP 0290761 A werden Polyetherblockcopolyamide beschrieben. Diese setzen sich aus Polyamiden mit Carboxylendgruppen und Polyethern zusammen. Als Polyether werden Polyetherdiole genannt.

EP 0577348 A offenbart Amidoamine aus Polyetheraminen und Lactamen. Zudem kann eine Dicarbonsäure hinzugefügt werden.

In US 2005/014842 A1 werden Polyetheramide genannt, welche aus der Reaktion von Dicarbonsäuren mit 6 bis 14 Kohlenstoffen, Polyetherdiaminen und linearenaliphatischen Diaminen mit 6 bis 14 C-Atomen erhalten werden.

DE 197 47 309 beschreibt die Verwendung eines Polyamid-12-Pulvers mit erhöhter

Schmelztemperatur und erhöhter Schmelzenthalpie, welches durch Umfällung eines zuvor durch Ringöffnung und anschließende Polykondensation von Laurinlactam hergestellten Polyamids erhalten wird. Es handelt sich dabei um ein Polyamid 12. Nachteilig an Bauteilen nach dem Stand der Technik ist eine schlechte Schlagzähigkeit. Diese ist ähnlich schlecht wie bei spritzgegossenen Polyamidbauteilen. Insbesondere, wenn Anwendungen, die über das Prototyping hinausgehen, wie beispielsweise Kleinserien, realisiert werden sollen, wird aber eine gute Schlagzähigkeit der Bauteile unabdingbar. Bei einem Einsatz im Automobilbereich müssen Bauteile auch bei tiefen Temperaturen noch ausreichende Schlagzähigkeiten aufweisen.

Nachteilig am Stand der Technik ist es ebenfalls, dass eine Übertragung der für Granulate gefundenen Schlagzähmodifikationen auf pulverförmige Werkstoffe nicht möglich ist. Entsprechend ausgerüstete Compounds sind in der Regel nicht vermahlbar oder nur in Ausbeuten, die einen kommerziellen Einsatz nicht realisieren lassen.

Aufgabe der vorliegenden Erfindung war es deshalb, ein Polymerpulver bereitzustellen, welches die Erzeugung von schlagzähen Formkörpern mit möglichst gut reproduzierbarem Verarbeitungsverfahren ermöglicht. Das Verarbeitungsverfahren ist dabei ein schichtweise arbeitendes Verfahren, bei dem selektiv Bereiche der jeweiligen Pulverschicht mittels elektromagnetischer Energie aufgeschmolzen werden und sich nach dem Abkühlen zu dem gewünschten Formkörper verbunden haben.

Überraschenderweise wurde nun gefunden, wie in den Ansprüchen beschrieben, dass sich durch die Verwendung von Blockpolyetheramid auf Basis von Oligoamiddicarbonsäuren und Polyetheraminen, bevorzugt Polyetherdiaminen, beispielsweise durch Polykondensation und anschließende Vermahlung Polymerpulver herstellen lassen, aus denen sich Formkörper durch ein schichtweise arbeitendes Verfahren, bei welchem selektiv Bereiche der jeweiligen Pulverschicht aufgeschmolzen werden, produzieren lassen, die Vorteile bezüglich der Schlagzähigkeit aufweisen, auch bei tiefen Temperaturen, und dabei vergleichbar gute Verarbeitungseigenschaften und mechanische Eigenschaften aufweisen wie aus einem Polymerpulver nach dem Stand der Technik, beispielsweise nach DE 197 47 309.

Gegenstand der vorliegenden Erfindung ist deshalb ein Polymerpulver zum Verarbeiten in einem schichtweise arbeitenden Verfahren, bei welchem selektiv Bereiche der jeweiligen Schicht aufgeschmolzen werden, welches dadurch gekennzeichnet ist, dass das Pulver zumindest ein Blockpolyetheramid bestehend aus Oligoamiddicarbonsäuren und Polyetheraminen, bevorzugt ein Blockpolyetheramid hergestellt durch Polykondensation von Oligoamiddicarbonsäuren und Polyetherdiaminen, aufweist.

Dabei weist das erfindungsgemäße Blockpolyetheramidpulver einen Schmelzpunkt zwischen 140 und 200 °C auf, eine Schmelzenthalpie zwischen 15 und 100 J/g sowie eine Rekristallisationstemperatur zwischen 50 und 190 °C. Bevorzugt liegt die Rekristallisationstemperatur so niedrig wie möglich.
Die verschiedenen Parameter wurden mittels DSC (Differential Scanning Calorimetry) nach DIN 53765, bzw. nach AN-SAA 0663 bestimmt. Die Messungen wurden mit einem Perkin Elmer DSC 7 mit Stickstoff als Spülgas und einer Aufheizrate sowie Abkühlrate von 20 K/min durchgeführt.

Die BET-Oberfläche liegt bei dem erfindungsgemäßen Blockpolyetheramid-Pulver kleiner als 5 m²/g, bevorzugt kleiner als 3 m²/g und besonders bevorzugt kleiner als 2 m²/g. Der mittlere Korndurchmesser liegt bevorzugt zwischen 40 und 120 µm, bevorzugt zwischen 45 und 100 µm und besonders bevorzugt zwischen 50 und 70 µm. Die Kornverteilung kann dabei eng, breit oder auch bimodal sein. Das Kornband liegt zwischen 0 und 180 µm, bevorzugt zwischen 0 und 120 µm, und besonders bevorzugt zwischen 0 und 100 µm. Die Schüttdichte liegt zwischen 300 g/l und 550 g/l (ohne Füllstoffe).

Die BET-Oberfläche wird durch Gasadsorption nach dem Prinzip von Brunauer, Emmet und Teller ermittelt; die herangezogene Norm ist die DIN ISO 9277.
Die Lösungsviskosität am Polyamid wird dabei nach DIN EN ISO 307 in 0,5%iger m-Kresollösung ermittelt.
Die Schüttdichte wurde mit einer Apparatur gemäß DIN 53 466 ermittelt.

Die Messwerte der Laserbeugung wurden an einem Malvern Mastersizer S, Ver. 2.18 erhalten.

Außerdem sind Gegenstand vorliegenden Erfindung Formkörper, hergestellt durch ein schichtweise arbeitendes Verfahren, welches selektiv Bereiche der jeweiligen Schicht aufschmilzt, welche dadurch gekennzeichnet sind, dass sie zumindest Blockpolyetheramid, aus Oligoamiddicarbonsäuren und Polyetheraminen, bevorzugt ein Blockpolyetheramid aus Oligoamiddicarbonsäuren und Polyetherdiaminen, und gegebenenfalls weitere Zuschlagstoffe, wie z. B. Stabilisatoren, Füllstoffe, Pigmente, Verlaufsmittel und Rieselhilfen, aufweisen.

Das erfindungsgemäße Blockpolyetheramidpulver hat den Vorteil, dass aus ihm durch ein schichtweise arbeitendes Verfahren, bei dem selektiv Bereiche der jeweiligen Schicht aufgeschmolzen werden, hergestellte Formkörper eine erhöhte Schlagzähigkeit gegenüber Formkörpern aus herkömmlichen Polyamidpulvern haben. Dabei weist das erfindungsgemäße Pulver eine vergleichbare Prozesssicherheit auf gegenüber herkömmlichen Polyamidpulver. Die Vermahlung ist deutlich einfacher und die Ausbeuten sind höher als bei der Vermahlung eines nicht erfindungsgemäßen PEBA, welches Polyetherester aufweist.

Die aus dem erfindungsgemäßen Pulver hergestellten Formkörper weisen dabei ähnlich gute mechanische Eigenschaften auf wie die aus herkömmlichen Polyamid 12-Pulver hergestellten Formkörper. Gegenüber letzteren weisen sie eine deutlich verbesserte Kerbschlagzähigkeit nach ISO 179 1eA auf, insbesondere bei tiefen Temperaturen. Auch die Reißdehnung ist meist verbessert. Der Elastizitätsmodul hingegen kann sowohl im Bereich des Standardmaterials liegen, als auch deutlich darunter. Er lässt sich durch die Zusammensetzung des Blockpolyetheramids einstellen. Daher ist die gezielte Einstellung für sehr flexible Bauteile, hergestellt aus erfindungsgemäßem Polymerpulver nach einem erfindungsgemäßen Verfahren, ebenso realisierbar wie die Herstellung relativ harter und schlagzäher Bauteile, gemessen am Standard-PA12-Polymerpulver. In einer bevorzugten Ausführungsform setzt man aminterminierte Blockpolyetheramide ein und erhält so weiter verbesserte mechanische Kennwerte an den Bauteilen.

Das erfindungsgemäße Blockpolyetheramidpulver zum Verarbeiten in einem schichtweise arbeitenden Verfahren, bei welchem selektiv Bereiche der jeweiligen Schicht aufgeschmolzen werden, zeichnet sich dadurch aus, dass das Pulver zumindest ein Blockpolyetheramid aus Oligoamiddicarbonsäuren und Polyetheraminen, bevorzugt ein Blockpolyetheramid aus Oligoamiddicarbonsäuren und Polyetherdiaminen, aufweist. Polyetheramide und ihre Herstellung sind prinzipiell aus der DE-OS 030 06 961 bekannt.

Zur Herstellung des Blockpolyetheramids werden Polyetheramin und die polyamidbildenden Ausgangsstoffe in einen geeigneten Poykondensationsreaktor nach dem Stand der Technik gegeben. Dabei können die Komponenten gleichzeitig oder auch zeitlich versetzt zugegeben werden. Die Komponenten werden unter Rühren unter Stickstoffatmosphäre erhitzt und anschließend so lange wie erforderlich gegebenenfalls unter Vakuum und Temperatur gehalten. Nach Erreichen der gewünschten Qualität wird das Polymere aus dem Reaktor gefahren und dabei als Strang granuliert. Eine Trocknung des Granulats, gegebenenfalls unter Stickstoffatmosphäre, kann angeschlossen werden.

Erfindungsgemäßes Blockpolyetheramidpulver erhält man durch Vermahlen, bevorzugt bei tiefen Temperaturen, besonders bevorzugt unterhalb 0 °C und ganz besonders bevorzugt unterhalb -25 °C, wobei ein Blockpolyetheramid aus Oligoamiddicarbonsäuren und Polyetheraminen, bevorzugt Polyetherdiaminen, als Ausgangsmaterial verwendet wird. Zum Vermahlen eignen sich unter anderem Stiftmühlen, Fließbettgegenstrahlmühlen oder Pralltellermühlen. Auch eine Nachbehandlung in einem Mischer mit starker Scherung, bevorzugt bei Temperaturen oberhalb des Glaspunktes des Polymeren, kann angeschlossen werden, um das Korn abzurunden und damit die Rieselfähigkeit zu verbessern. Auch eine Fraktionierung, beispielsweise durch Sieben oder Sichten, kann die Pulvereigenschaften verbessern. Eine Ausrüstung mit Rieselhilfen nach dem Stand der Technik kann ebenfalls angeschlossen werden. Überraschenderweise lässt sich mit diesen Maßnahmen ein gut verarbeitbares Pulver erzeugen, was eine sichere und kommerziell nutzbare Verarbeitung mit einem erfindungsgemäßen Verfahren ermöglicht.

Überraschenderweise wurde festgestellt, dass die Nachteile, insbesondere die schlechte Vermahlbarkeit von schlagzäh ausgerüstetem Granulat sich bei Pulver mit dem erfindungsgemäßen Blockpolyetheramid aus Oligoamiddicarbonsäuren und Polyetheraminen, nicht zeigen. Die Vermahlung bei tiefen Temperaturen ist ohne weiteres möglich, wobei die Ausbeuten im kommerziell nutzbaren Bereich liegen. Beim Verarbeiten in einem der beschriebenen werkzeuglosen Herstellverfahren können dabei Bauteile hergestellt werden, die eine Kerbschlagzähigkeit nach ISO 179 1eA bei Raumtemperatur als auch bei -30 °C von mehr als 15 kJ/m² aufweisen, bevorzugt mehr als 25 kJ/m².
Der Elastizitätsmodul kann dabei zwischen 50 N/mm² und mehr als 2000 N/mm² liegen. Je nach Zusammensetzung kann dabei ein sehr flexibles Material hergestellt werden, beispielsweise mit einem Elastizitätsmodul an einem daraus mit einem erfindungsgemäßen Verfahren hergestellten Zugstab zwischen 50 und 600 N/mm² nach ISO 527, oder ein Material mit höherer Steifigkeit, beispielsweise mit einem Elastizitätsmodul an einem daraus mit einem erfindungsgemäßen Verfahren hergestellten Zugstab zwischen 600 und 2000 N/mm² nach ISO 527. Die Dichte der mit einem erfindungsgemäßen Verfahren hergestellten Bauteile beträgt dabei mehr als 0,88 g/cm³, bevorzugt mehr als 0,9 g/cm³, und besonders bevorzugt mehr als 0,92 g/cm³.

Die verwendeten Polyetheramine haben primäre Aminogruppen und einen Rücken aus Polyethereinheiten. Der Polyetherrücken kann beispielsweise auf Propylenoxid, Ethylenoxid, Polytetramethylenoxid, oder einer Mischung aus zwei oder allen der vorgenannten bestehen. Die einzelnen Ethereinheiten sind bevorzugt alkylverzweigt. Es kann sich bei den Polyetheraminen im Mono, Di-, oder Triamine handeln, wobei Diamine besonders bevorzugt sind. Das Molekulargewicht (Gewichtsmittel) liegt zwischen 200 und 5000 g/mol. Die Polyetheramine bilden den Weichblock in dem Copolymer.
Kommerziell erhältliche Produkte sind die Polyetheramine der D-Reihe der BASF AG, Deutschland, beispielsweise Polyetheramin D400, aber auch die Jeffamin-Reihe der Huntsman Corp., Texas, beispielsweise Jeffamin D2000.

Die verwendeten Oligoamiddicarbonsäuren liegen im Molekulargewicht zwischen 1000 und 20000 g/mol. Die Oligoamiddicarbonsäuren bilden den Hartblock im Copolymer. Für die weichen Einstellungen wird die Länge des Hartblockes bevorzugt unterhalb von 1500 g/mol gewählt, für die festen Einstellungen beträgt die Länge des Hartblockes bevorzugt mehr als 5000 g/mol; beide Angaben beziehen sich auf die Verwendung eines linearen diaminterminierten Polyethers.
Die Oligoamiddicarbonsäure wird beispielsweise erhalten aus Laurinlactam oder einem anderen Lactam mit 8 oder mehr C-Atomen beziehungsweise der korrespondierenden ω-Aminocarbonsäuren und einer Dicarbonsäure, vorzugsweise einer linearen aliphatischen Dicarbonsäure, besonders bevorzugt Dodecandisäure. Ebenso können Oligoamiddicarbonsäuren aus aliphatischen Diaminen mit überschüssiger, aliphatischer Dicarbonsäure mit den genannten Polyetheraminen kondensiert werden.

Bei der Polykondensation ist es vorteilhaft, einen Katalysator hinzuzugeben, beispielsweise unterphosphorige Säure. Es können auch Stabilisatoren sowie Costabilisatoren nach dem Stand der Technik zugegeben werden; beispielhaft genannt werden sollen sterisch gehinderte Phenole oder Phosphite. Die Lösungsviskosität des Blockpolyetheramiden wird über den Prozess und die Zugabe des Katalysators eingestellt und kann zwischen 1,4 und 2,1 liegen, bevorzugt zwischen 1,5 und 1,9, und besonders bevorzugt zwischen 1,6 und 1,8. Bei der Polykondensation erhält man ein Blockpolyetheramid, wobei die Polyamidkomponente den Hartblock bildet und die Polyetheraminkomponente den Weichblock. Je nach zugegebenen Anteilen der beiden Komponenten im Reaktor erhält man Material mit einem Überschuss an Aminoendgruppen oder Säureendgruppen, oder auch ein Material mit gleicher Anzahl beider Endgruppen. Bevorzugt weist das Blockpolyetheramid einen Überschuss an Aminoendgruppen auf. Die Anzahl von Aminoendgruppen zu Carboxylendgruppen sollte um nicht mehr als 10 % abweichen.

Dabei weist das erfindungsgemäße Blockpolyetheramidpulver einen Schmelzpunkt zwischen 140 und 200 °C auf, eine Schmelzenthalpie zwischen 15 und 100 J/g sowie eine Rekristallisationstemperatur zwischen 50 und 190 °C. Bevorzugt liegt die Rekristallisationstemperatur so niedrig wie möglich.

Der Glasübergang hängt vom jeweiligen Polyetheramin ab und liegt bei Verwendung eines linearen Polyetheramins mit einem Molekulargewicht von ca. 2000 g/mol beispielsweise bei -60 °C und bei Verwendung eines linearen Polyetheramins mit einem Molekulargewicht von ca. 400 g/mol beispielsweise bei -12 °C. Je nach Länge des Polyamid-Hartblocks findet man oft einen zweiten Glasübergang, der niedriger als der des reinen entsprechenden Polyamids liegt. Beispielsweise ist bei Hartblöcken kleiner als 2000 g/mol meist kein zweiter Glasübergang zu entdecken, bei Hartblöcken, bestehend aus Laurinlactam und Dodecandisäure, größer als 2500 g/mol ist ein Glasübergang bei ca. 27 °C festzustellen, der sich mit steigender Hartblocklänge in Richtung des Glasübergangs des reinen Polyamids, in diesem Fall des Polyamid 12 bei 38 °C, bewegt.

Zur definierten Einstellung von hartem Material, aus welchem sich Bauteile herstellen lassen, die auch bei Temperaturen von -30 °C eine Kerbschlagzähigkeit nach ISO 179 1eA größer als 10 kJ/m² zeigen und gleichzeitig einen Elastizitätsmodul nach ISO 527 größer als 600 N/mm² haben, besteht eine vorteilhafte Ausführung in der Aufteilung des Polyetheraminanteils in Polyetheramine unterschiedlichen Molekulargewichtes. Eine Aufteilung des Polyetheraminanteils mit Molekulargewicht 400 g/mol zu 2000 g/mol von 1:1, 2:1, oder 1:2 hat sich dabei als bevorzugte Ausführung erwiesen.

Die BET-Oberfläche nach dem Prinzip von Brunauer, Emmet. Teller, DIN ISO 9277, ist bei dem erfindungsgemäßen Blockpolyetheramidpulver kleiner als 5 m²/g, bevorzugt kleiner als 3 m²/g und besonders bevorzugt kleiner als 2 m²/g. Der mittlere Korndurchmesser liegt bevorzugt zwischen 40 und 120 µm, bevorzugt zwischen 45 und 100 µm und besonders bevorzugt zwischen 50 und 70 µm. Das Kornband liegt zwischen 0 und 180 µm, bevorzugt zwischen 0 und 120 µm, und besonders bevorzugt zwischen 0 und 100 µm. Die Kornverteilung kann dabei eng, breit, oder auch bimodal sein. Die Schüttdichte liegt zwischen 300 g/l und 500 g/l (ohne Füllstoffe).
Die Lösungsviskosität in 0,5-%iger m-Kresollösung nach DIN EN ISO 307 beträgt bei den erfindungsgemäßen Blockpolyetheramidpulvern bevorzugt 1,4 bis 2,1, besonders bevorzugt 1,5 bis 1,9 und ganz besonders bevorzugt zwischen 1,6 und 1,8.

Erfindungsgemäßes Blockpolyetheramidpulver kann außerdem Hilfsstoffe und/oder Füllstoffe und/oder weitere organische oder anorganische Pigmente aufweisen. Solche Hilfsstoffe können z. B. Rieselhilfsmittel, wie z. B. gefällte und/oder pyrogene Kieselsäuren, sein. Gefällte Kieselsäuren werden zum Beispiel unter dem Produktnamen Aerosil, mit unterschiedlichen Spezifikationen, durch die Degussa AG angeboten. Vorzugsweise weist erfindungsgemäßes Polymerpulver weniger als 3 Gew.-%, vorzugsweise von 0,001 bis 2 Gew.-% und ganz besonders bevorzugt von 0,05 bis 1 Gew.-% solcher Hilfsstoffe, bezogen auf die Summe der vorhandenen Polymere, auf. Die Füllstoffe können z. B. Glas-, Metall- oder Keramikpartikel, wie z. B. Glaskugeln, Stahlkugeln oder Metallgrieß, oder Fremdpigmente, wie z. B. Übergangsmetalloxide, sein. Die Pigmente können beispielsweise Titandioxidpartikel basierend auf Rutil (bevorzugt) oder Anatas sein oder Rußpartikel.

Die Füllstoffpartikel weisen dabei vorzugsweise eine kleinere oder ungefähr gleich große mittlere Partikelgröße wie die Partikel der Blockpolyetheramide auf. Vorzugsweise sollte die mittlere Partikelgröße d₅₀ der Füllstoffe die mittlere Partikelgröße d₅₀ der Blockpolyetheramide um nicht mehr als 20 %, vorzugsweise um nicht mehr als 15 % und ganz besonders bevorzugt um nicht mehr als 5 % überschreiten. Die Partikelgröße ist insbesondere limitiert durch die zulässige Bauhöhe bzw. Schichtdicke in der Rapid-Prototyping/Rapid Manufacturing-Anlage.

Vorzugsweise weist erfindungsgemäßes Polymerpulver weniger als 75 Gew.-%, bevorzugt von 0,001 bis 70 Gew.-%, besonders bevorzugt von 0,05 bis 50 Gew.-% und ganz besonders bevorzugt von 0,5 bis 25 Gew.-% solcher Füllstoffe, bezogen auf die Summe der vorhandenen Blockpolyetheramide, auf.

Beim Überschreiten der angegebenen Höchstgrenzen für Hilfs- und/oder Füllstoffe kann es je nach eingesetztem Füll- oder Hilfsstoff zu deutlichen Verschlechterungen der mechanischen Eigenschaften von Formkörpern kommen, die mittels solcher Polymerpulver hergestellt wurden.

Es ist ebenso möglich, herkömmliche Polymerpulver mit erfindungsgemäßen Polymerpulvern zu mischen. Auf diese Weise lassen sich Polymerpulver in einem weiten Bereich bezüglich der Flexibilität und der Schlagzähigkeit herstellen. Das Verfahren zur Herstellung solcher Mischungen kann z. B. DE 34 41 708 entnommen werden.

Zur Verbesserung des Schmelzeverlaufs bei der Herstellung der Formkörper kann ein Verlaufsmittel, wie beispielsweise Metallseifen, bevorzugt Alkali- oder Erdalkalisalze der zugrunde liegenden Alkanmonocarbonsäuren oder Dimersäuren, dem Blockpolyetheramidpulver zugesetzt werden. Die Metallseifenpartikel können in die Polymerpartikel eingearbeitet werden, es können aber auch Mischungen von feinteiligen Metallseifenpartikeln und Polymerpartikeln vorliegen.

Die Metallseifen werden in Mengen von 0,01 bis 30 Gew.-%, vorzugsweise 0,5 bis 15 Gew.-%, bezogen auf die Summe der im Pulver vorhandenen Blockpolyetheramide, eingesetzt. Bevorzugt wurden als Metallseifen die Natrium- oder Calciumsalze der zugrunde liegenden Alkanmonocarbonsäuren oder Dimersäuren eingesetzt. Beispiele für kommerziell verfügbare Produkte sind Licomont NaV 101 oder Licomont CaV 102 der Firma Clariant.

Zur Verbesserung der Verarbeitungsfähigkeit oder zur weiteren Modifikation des Polymerpulvers können diesem anorganische Fremdpigmente, wie z. B. Übergangsmetalloxide, Stabilisatoren, wie z. B. Phenole, insbesondere sterisch gehinderte Phenole, Verlaufs- und Rieselhilfsmittel, wie z. B. pyrogene Kieselsäuren, sowie Füllstoffpartikel zugegeben werden. Vorzugsweise wird, bezogen auf das Gesamtgewicht an Polymeren im Polymerpulver, so viel dieser Stoffe den Polymeren zugegeben, dass die für das erfindungsgemäße Polymerpulver angegeben Konzentrationen für Füll- und/oder Hilfsstoffe eingehalten werden.

Gegenstand der vorliegenden Erfindung sind auch Verfahren zur Herstellung von Formkörpern durch schichtweise arbeitende Verfahren, bei denen selektiv Bereiche der jeweiligen Schicht aufgeschmolzen werden, die erfindungsgemäße Polymerpulver, welche dadurch gekennzeichnet sind, dass sie zumindest ein Blockpolyetheramid aus einer Oligoamiddicarbonsäure und einem Polyetheramin, bevorzugt Polyetherdiamin, aufweisen.

Die Energie wird durch elektromagnetische Strahlung eingebracht, und die Selektivität wird beispielsweise durch Masken, Auftragung von Inhibitoren, Absorbern, Suszeptoren oder aber durch eine Fokussierung der Strahlung, beispielsweise durch Laser, eingebracht. Die elektromagnetische Strahlung umfasst den Bereich von 100 nm bis 10 cm, bevorzugt zwischen 400 nm bis 10 600 nm und besonders bevorzugt 10600 nm (CO2-Laser) oder 800 bis 1 060 nm (Diodenlaser, Nd:YAG-Laser, oder entsprechende Lampen und Strahler). Die Quelle der Strahlung kann beispielsweise ein Mikrowellengenerator, ein geeigneter Laser, ein Heizstrahler oder eine Lampe sein, aber auch Kombinationen davon. Nach dem Abkühlen aller Schichten kann der erfindungsgemäße Formkörper entnommen werden. Es kann vorteilhaft sein, den Bauraum der Maschine zu temperieren.

Die nachfolgenden Beispiele für solche Verfahren dienen der Erläuterung, ohne die Erfindung darauf beschränken zu wollen.

Die Lasersinterverfahren sind hinlänglich bekannt und beruhen auf dem selektiven Sintern von Polymerpartikeln, wobei Schichten von Polymerpartikeln kurz einem Laserlicht ausgesetzt werden und so die Polymerpartikel, die dem Laserlicht ausgesetzt waren, miteinander verbunden werden. Durch die aufeinander folgende Versinterung von Schichten von Polymerpartikeln werden dreidimensionale Objekte hergestellt. Einzelheiten zum Verfahren des selektiven Laser-Sinterns sind z. B. den Schriften US 6 136 948 und WO 96/06881 zu entnehmen.

Das erfindungsgemäße Pulver kann auch zum Verarbeiten in einem erfindungsgemäßen Verfahren verwendet werden, bei dem von Schicht zu Schicht oder auch innerhalb einer Schicht unterschiedliche Pulver verwendet werden. Beispielsweise kann auf diese Art und Weise ein Formkörper entstehen, der harte und weiche Bereiche aufweist.
Andere gut geeignete Verfahren sind das SIV-Verfahren, wie es in WO 01/38061 beschrieben wird, oder ein Verfahren wie in EP 1 015 214 beschrieben. Beide Verfahren arbeiten mit einer flächigen Infrarotheizung zum Aufschmelzen des Pulvers. Die Selektivität des Aufschmelzens wird bei ersterem durch die Auftragung eines Inhibitors, beim zweiten Verfahren durch eine Maske erreicht. Ein weiteres Verfahren ist in DE 103 11 438 beschrieben. Bei diesem wird die zum Verschmelzen benötigte Energie durch einen Mikrowellengenerator eingebracht, und die Selektivität wird durch Auftragen eines Suszeptors erreicht.

Weitere geeignete Verfahren sind solche, die mit einem Absorber arbeiten, der entweder im Pulver enthalten ist, oder der per Inkjet-Verfahren aufgetragen wird wie in DE 10 2004 012 682.8, DE 10 2004 012 683.6 und DE 10 2004 020 452.7 beschrieben.

Um optimale Ergebnisse zu erhalten, müssen das Blockpolyetheraminpulver und das verwendete Verfahren aufeinander abgestimmt werden. So kann es für Pulverauftragssysteme, die sich der Schwerkraft bedienen, von Vorteil sein, die Rieselfähigkeit des Pulvers mit geeigneten Maßnahmen nach dem Stand der Technik zu erhöhen. Eine Vorheizung der Baukammer, oder auch des Pulvers kann positiv für die Verarbeitbarkeit und die Bauteilqualität sein. Gute Ergebnisse wurden auch damit erzielt, dass die ersten Schichten eines Bauteils mit anderem, meist höherem, Energieeintrag behandelt wurden als die nachfolgenden. Die Einstellungsmöglichkeiten beispielsweise bezüglich Leistung, Einwirkdauer, Frequenz der elektromagnetischen Strahlung sind vielfältig und werden hier nicht vollständig aufgeführt; sie können aber von dem Fachmann in Vorversuchen einfach ermittelt werden.

Die erfindungsgemäßen Formkörper, die durch ein schichtweise arbeitendes Verfahren, bei dem selektiv Bereiche aufgeschmolzen werden, hergestellt werden, zeichnen sich dadurch aus, dass sie zumindest ein Blockpolyetheramid, aus einem Polyetheramin, bevorzugt Polyetherdiamin, und eine Oligoamiddicarbonsäure, aufweisen.

Die Formkörper können außerdem Füllstoffe und/oder Hilfsstoffe (hier gelten die Angaben wie für das Polymerpulver), wie z. B. thermische Stabilisatoren, wie z. B. sterisch gehinderte Phenolderivate, aufweisen. Füllstoffe können z. B. Glas-, Keramikpartikel und auch Metallpartikel, wie zum Beispiel Eisenkugeln, bzw. entsprechende Hohlkugeln sein. Bevorzugt weisen die erfindungsgemäßen Formkörper Glaspartikel, ganz besonders bevorzugt Glaskugeln auf. Vorzugsweise weisen erfindungsgemäße Formkörper weniger als 3 Gew.-%, besonders bevorzugt von 0,001 bis 2 Gew.-% und ganz besonders bevorzugt von 0,05 bis 1 Gew.-% solcher Hilfsstoffe, bezogen auf die Summe der vorhandenen Polymere, auf. Ebenso bevorzugt weisen erfindungsgemäße Formkörper weniger als 75 Gew.-%, bevorzugt von 0,001 bis 70 Gew.-%, besonders bevorzugt von 0,05 bis 50 Gew.-% und ganz besonders bevorzugt von 0,5 bis 25 Gew.-% solcher Füllstoffe, bezogen auf die Summe der vorhandenen Polymere, auf.

Die erfindungsgemäßen Formkörper zeichnen sich durch eine sehr gute Schlagzähigkeit bzw. Kerbschlagzähigkeit aus, vor allem bei tiefen Temperaturen. So sind Kerbschlagzähigkeiten nach DIN EN ISO 179 1eA von 15 kJ/m², sowohl bei Raumtemperatur als auch bei -30 °C problemlos zu erreichen, aber auch Werte von mehr als 20 kJ/m² oder auch mehr als 40 kJ/m², werden je nach Zusammensetzung des Blockpolyetheramiden ohne weiteres erreicht. Vorrausgesetzt, daß die Bauteile nicht zu viele Lunker aufweisen, oder eine Dichte größer 0,9 g/mm³ aufweisen, kann man beobachten, dass die Kerbschlagzähigkeiten bei -30 °C sogar höher sind als bei Raumtemperatur. Die Reißdehnungen nach ISO 527 liegen in der Regel oberhalb von 30 %, aber meist werden noch deutlich höhere Werte gemessen.

Die Lösungsviskosität am erfindungsgemäßen Bauteil in 0,5-%iger m-Kresollösung nach DIN EN ISO 307 kann im Bereich von 20 % Absenkung bis zu 50 % Steigerung gegenüber der Lösungsviskosität am verwendeten Blockpolyetheraminpulver liegen. Bevorzugt liegt sie im Bereich von 10 % Absenkung bis zu 30 % Steigerung gegenüber der Lösungsviskosität am verwendeten Blockpolyetheraminpulver. Besonders bevorzugt findet ein Anstieg der Lösungsviskosität während des erfindungsgemäßen Bauprozesses statt.
Der Elastizitätsmodul, gemessen am erfindungsgemäßen Formteil, kann dabei zwischen 50 N/mm² und mehr als 2000 N/mm² liegen. Je nach Zusammensetzung des verwendeten Blockpolyetheraminpulvers kann dabei ein sehr flexibles Formteil hergestellt werden, beispielsweise mit einem Elastizitätsmodul an einem daraus mit einem erfindungsgemäßen Verfahren hergestellten Zugstab zwischen 50 und 600 N/mm² nach ISO 527, oder ein Formteil mit höherer Steifigkeit, beispielsweise mit einem Elastizitätsmodul an einem daraus mit einem erfindungsgemäßen Verfahren hergestellten Zugstab zwischen 600 und 2000 N/mm² nach ISO 527. Die Dichte der mit einem erfindungsgemäßen Verfahren hergestellten Bauteile beträgt dabei mehr als 0,88 g/mm³, bevorzugt mehr als 0,9 g/mm³, und besonders bevorzugt mehr als 0,92 g/mm³.

Anwendungsgebiete für diese Formkörper sind sowohl im Rapid Prototyping als auch im Rapid Manufacturing zu sehen. Mit letzterem sind durchaus Kleinserien gemeint, also die Herstellung von mehr als einem gleichen Teil, bei dem aber die Produktion mittels eines Spritzgießwerkzeugs nicht wirtschaftlich ist. Beispiele hierfür sind Teile für hochwertige PKW, die nur in kleinen Stückzahlen hergestellt werden, oder Ersatzteile für den Motorsport, bei denen neben den kleinen Stückzahlen auch der Zeitpunkt der Verfügbarkeit eine Rolle spielt. Branchen, in die die erfindungsgemäßen Teile gehen, können die Luft- und Raumfahrtindustrie sein, die Medizintechnik, der Maschinenbau, der Automobilbau, die Sportindustrie, die Haushaltswarenindustrie, Elektroindustrie und Lifestyle.

Die folgenden Beispiele sollen das erfindungsgemäße Polymerpulver sowie dessen Verwendung beschreiben, ohne die Erfindung auf die Beispiele einzuschränken.

### Beispiele

### Vergleichsbeispiel 1:

EOSINT PPA2200, Standardmaterial für das Lasersintern, welches beispielsweise bei der EOS GmbH in Krailling, Deutschland, bezogen werden kann.

### Vergleichsbeispiel 2:

Zur Herstellung eines PEBA basierend auf PA12 mit Hartblock von 1062 dalton und äqumolarer Mengen PTHF 1000 und PTHF 2000 wurde eine 200 I-Zweikessel-Polykondensationsanlage - bestehend aus Ansatzbehälter mit Ankerrührer und Polykondensationsreaktor mit Wendelrührer - mit folgenden Einsatzstoffen beschickt:
1. Chargierung:

| | |
|---|---|
| 34,418 kg | Laurinlactam, |
| 8,507 kg | Dodecandisäure, |

sowie
2.Chargierung

| | |
|---|---|
| 38,050 kg | PTHF 2000, |
| 19,925 kg | PTHF 1000 |
| 43,0 g | einer 50-%igen wässrigen Lösung von Hypophosphoriger Säure (entspricht 0,05 Gew.-%). |

Die Einsatzstoffe der 1. Chargierung wurden in einer Stickstoffatmosphäre bei 180°C aufgeschmolzen, in den Polykondensationsreaktor gedrückt und unter Rühren im geschlossenen Autoklaven 6 Stunden auf ca. 280 °C erhitzt. Währenddessen wurde im Ansatzbehälter die 2. Chargierung auf 180°C vorgewärmt und zur Oligoamid-Dicarbonsäureschmelze im Polykondensationsreaktor gedrückt. Nach Entspannen auf Normaldruck wird diese Mischung bei 238°C ca. 5 Stunden im Stickstoffstrom unter Rühren bei dieser Temperatur gehalten. Anschließend wurde innerhalb von 3 Stunden ein Vakuum von 200 mbar angelegt und bis zum Erreichen des gewünschten Drehmoments gehalten. Danach wurde die Schmelze unter 10 bar Stickstoffdruck gesetzt und mittels Zahnradpumpe ausgetragen und als Strang granuliert. Das Granulat wurde 24 Stunden unter Stickstoff bei 80 °C getrocknet.
Austrag: 96 kg

Das Produkt wies folgende Kennwerte auf:

| | |
|---|---|
| Kristallitschmelzpunkt Tₘ: | 150 °C |
| Relative Lösungsviskosität ηᵣₑₗ: | 2,12 |

COOH-Endgruppen: 43mmol/kg

### Vergleichsbeispiel 3:

Ein Standardprodukt der Degussa AG, Marl, nämlich Vestamid E40 S3 wird kalt vermahlen. Dabei handelt es sich um ein Polyetheresterblockamid mit einem Weichblock aus Polytetrahydrofuran 1000 und einer Shore-Härte von 40 D.

### Vergleichsbeispiel 4:

Ein Standardprodukt der Degussa AG, Marl, nämlich Vestamid E55 S3 wird kalt vermahlen. Dabei handelt es sich um ein Polyetheresterblockamid mit einem Weichblock aus Polytetrahydrofuran 1000 und einer Shore-Härte von 55 D.

### Beispiel 1:

Zur Herstellung eines PEA basierend auf PA12 mit Hartblock von 2392 dalton und Jeffamin D2000 wurde eine 200 I-Zweikessel-Polykondensationsanlage - bestehend aus Ansatzbehälter mit Ankerrührer und Polykondensationsreaktor mit Wendelrührer - mit folgenden Einsatzstoffen beschickt:
1. Chargierung:

| | |
|---|---|
| 45,186 kg | Laurinlactam, |
| 4,814 kg | Dodecandisäure, sowie |

2.Chargierung

| | |
|---|---|
| 43,060 kg | Jeffamine D2000, |
| 93,0 g | einer 50 %igen wässrigen Lösung von Hypophosphoriger Säure (entspricht 0,05 Gew.-%). |

Die Einsatzstoffe der 1. Chargierung wurden in einer Stickstoffatmosphäre bei 180°C aufgeschmolzen, in den Polykondensationsreaktor gedrückt und unter Rühren im geschlossenen Autoklaven 6 Stunden auf ca. 280 °C erhitzt. Währenddessen wurde im Ansatzbehälter die 2. Chargierung auf 180°C vorgewärmt und zur Oligoamid-Dicarbonsäureschmelze im Polykondensationsreaktor gedrückt. Nach Entspannen auf Normaldruck wird diese Mischung bei 220°C ca. 5 Stunden im Stickstoffstrom unter Rühren bei dieser Temperatur gehalten. Anschließend wurde innerhalb von 2 Stunden ein Vakuum von 100 mbar angelegt und bis zum Erreichen des gewünschten Drehmoments gehalten. Danach wurde die Schmelze unter 10 bar Stickstoffdruck gesetzt und mittels Zahnradpumpe ausgetragen und als Strang granuliert. Das Granulat wurde 24 Stunden unter Stickstoff bei 80 °C getrocknet.
Austrag: 92kg

Das Produkt wies folgende Kennwerte auf:

| | |
|---|---|
| Kristallitschmelzpunkt Tₘ: | 167 °C |
| Relative Lösungsviskosität ηᵣₑₗ: | 1,66 |

| | |
|---|---|
| COOH-Endgruppen: 48 mmol/kg | NH₂-Endgruppen : 17 mmol/kg |

### Beispiel 2:

Zur Herstellung eines PEA basierend auf PA12 mit Hartblock von 808 dalton und Jeffamin D400 wurde eine100 I-Zweikessel-Polykondensationsanlage - bestehend aus Ansatzbehälter mit Ankerrührer und Polykondensationsreaktor mit Wendelrührer - mit folgenden Einsatzstoffen beschickt:
1. Chargierung:

| | |
|---|---|
| 46,473 kg | Laurinlactam, |
| 18,527 kg | Dodecandisäure, sowie |

2.Chargierung

| | |
|---|---|
| 37,949 kg | Jeffamine D400, |
| 100,0 g | einer 50 %igen wässrigen Lösung von Hypophosphoriger Säure (entspricht 0,05 Gew.-%). |

Die Einsatzstoffe der 1. Chargierung wurden in einer Stickstoffatmosphäre bei 180°C aufgeschmolzen, in den Polykondensationsreaktor gedrückt und unter Rühren im geschlossenen Autoklaven 6 Stunden auf ca. 280 °C erhitzt. Währenddessen wurde im Ansatzbehälter die 2. Chargierung auf 180°C vorgewärmt und zur Oligoamid-Dicarbonsäureschmelze im Polykondensationsreaktor gedrückt. Nach Entspannen auf Normaldruck wird diese Mischung bei 230°C ca. 5 Stunden im Stickstoffstrom unter Rühren bei dieser Temperatur gehalten. Anschließend wurde innerhalb von 2 Stunden ein Vakuum von 100 mbar angelegt und bis zum erreichen des gewünschten Drehmoments gehalten. Danach wurde die Schmelze unter 10 bar Stickstoffdruck gesetzt und mittels Zahnradpumpe
ausgetragen und als Strang granuliert. Das Granulat wurde 24 Stunden unter Stickstoff bei 80 °C getrocknet.
Austrag: 98kg

Das Produkt wies folgende Kennwerte auf:

| | |
|---|---|
| Kristallitschmelzpunkt Tₘ: | 135 °C |
| Relative Lösungsviskosität ηᵣₑₗ: | 1,60 |

| | |
|---|---|
| COOH-Endgruppen: 2 mmol/kg | NH₂ - Endgruppen : 76 mmol/kg |

### Beispiel 3:

Zur Herstellung eines PEBA basierend auf PA12 mit Hartblock von 2908 dalton und Jeffamin D2000 wurde das Beispiel 1 mit folgenden Einwaagen wiederholt:
1. Chargierung:

| | |
|---|---|
| 55,248 kg | Laurinlactam, |
| 4,752 kg | Dodecandisäure, sowie |

2.Chargierung

| | |
|---|---|
| 42,503 kg | Jeffamine D2000, |
| 101,0 g | einer 50 %igen wässrigen Lösung von Hypophosphoriger Säure (entspricht 0,05 Gew.-%). |

Austrag: 99 kg

Das Produkt zeigte folgende Kennwerte:

| | |
|---|---|
| Kristallitschmelzpunkt Tₘ: | 168 °C |
| Relative Lösungsviskosität ηᵣₑₗ: | 1,75 |

| | |
|---|---|
| COOH-Endgruppen: 19 mmol/kg | NH₂ - Endgruppen : 44 mmol/kg |

### Beispiel 4:

Zur Herstellung eines PEBA basierend auf PA12 mit Hartblock von 1068 dalton und Jeffamin D2000 wurde das Beispiel 1 - in einer 100I-Zweikesselanlage -mit folgenden Einwaagen wiederholt:
1. Chargierung:

| | |
|---|---|
| 12,172 kg | Laurinlactam, |
| 3,346 kg | Dodecandisäure, sowie |

2.Chargierung

| | |
|---|---|
| 28,430 kg | Jeffamine D2000, |
| 44,0 g | einer 50 %igen wässrigen Lösung von Hypophosphoriger Säure (entspricht 0,05 Gew.-%). |

Austrag: 41 kg

Das Produkt zeigte folgende Kennwerte:

| | |
|---|---|
| Kristallitschmelzpunkt Tₘ: | 150 °C |
| Relative Lösungsviskosität ηᵣₑₗ: | 1,63 |

| | |
|---|---|
| COOH-Endgruppen: 14mmol/kg | NH₂ - Endgruppen : 37 mmol/kg |

### Beispiel 5:

Zur Herstellung eines PEBA basierend auf PA12 mit Hartblock von 1068 dalton und Jeffamin D2000 wurde das Beispiel 4 - in einer 200I-Zweikesselanlage -mit folgenden Einwaagen wiederholt:
1. Chargierung:

| | |
|---|---|
| 27,453 kg | Laurinlactam, |
| 7,547 kg | Dodecandisäure, sowie |

2.Chargierung

| | |
|---|---|
| 67,509 kg | Jeffamine D2000, |
| 100,0 g | einer 50 %igen wässrigen Lösung von Hypophosphoriger Säure (entspricht 0,05 Gew.-%). |

Austrag: 91 kg

Das Produkt zeigte folgende Kennwerte:

| | |
|---|---|
| Kristallitschmelzpunkt Tₘ: | 151 °C |
| Relative Lösungsviskosität ηᵣₑₗ: | 1,63 |

| | |
|---|---|
| COOH-Endgruppen: 14 mmol/kg | NH₂ - Endgruppen : 37 mmol/kg |

### Beispiel 6-8:

Zur Herstellung eines PEBA basierend auf PA12 mit Hartblock von 1068 dalton und Jeffamin D2000 wurde das Beispiel 5 mit folgenden Einwaagen wiederholt:
1. Chargierung:

| | |
|---|---|
| 28,797 kg | Laurinlactam, |
| 7,547 kg | Dodecandisäure, sowie |

2.Chargierung

| | |
|---|---|
| 67,509 kg | Jeffamine D2000, |
| 100,0 g | einer 50 %igen wässrigen Lösung von Hypophosphoriger Säure (entspricht 0,05 Gew.-%). |

**Tabelle 1**

| Beispiel | Austrag kg | Tₘ °C | η ᵣₑₗ | COOH mmol/kg | NH₂ mmol/kg |
|---|---|---|---|---|---|
| 6 | 95 | 152 | 1,73 | 54 | 9 |
| 7 | 92 | 153 | 1,60 | 11 | 42 |
| 8 | 97 | 153 | 1,60 | 13 | 47 |

### Beispiel 9:

Zur Herstellung eines PEBA basierend auf PA12 mit Hartblock von 7225 dalton und Jeffamin D400 wurde das Beispiel 2 - in einer 100I Zweikesselanlage - mit folgenden Einwaagen wiederholt:
1. Chargierung:

| | |
|---|---|
| 43,566 kg | Laurinlactam, |
| 1,434 kg | Dodecandisäure, sowie |

2.Chargierung

| | |
|---|---|
| 2,938 kg | Jeffamine D400, |
| 47,0 g | einer 50 %igen wässrigen Lösung von Hypophosphoriger Säure (entspricht 0,05 Gew.-%). |

Austrag: 44 kg

Das Produkt zeigte folgende Kennwerte:

| | |
|---|---|
| Kristallitschmelzpunkt Tₘ: | 174 °C |
| Relative Lösungsviskosität ηᵣₑₗ: | 2,04 |

| | |
|---|---|
| COOH-Endgruppen: 32 mmol/kg | NH₂ - Endgruppen : 20 mmol/kg |

### Beispiel 10:

Zur Herstellung eines PEBA basierend auf PA12 mit Hartblock von 13000 dalton und Jeffamin D2000 wurde das Beispiel 9 mit folgenden Einwaagen wiederholt:
1. Chargierung:

| | |
|---|---|
| 42,238 kg | Laurinlactam, |
| 0,762 kg | Dodecandisäure, sowie |

2.Chargierung

| | |
|---|---|
| 6,814 kg | Jeffamine D2000, |
| 50,0 g | einer 50 %igen wässrigen Lösung von Hypophosphoriger Säure (entspricht 0,05 Gew.-%). |

Austrag: 44 kg

Das Produkt zeigte folgende Kennwerte:

| | |
|---|---|
| Kristallitschmelzpunkt Tₘ: | 176 °C |
| Relative Lösungsviskosität ηᵣₑₗ: | 1,73 |

| | |
|---|---|
| COOH-Endgruppen: 68 mmol/kg | NH₂ - Endgruppen : 60 mmol/kg |

### Vermahlung der Granulate:

Die Vermahlung der nicht erfindungsgemäßen Beispiele 2 bis 4 war deutlich schwieriger als die der erfindungsgemäßen Granulate. So musste die Temperatur auf - 70 °C abgesenkt werden, um Ausbeuten zu erhalten, die noch unter 50 % lagen. Bei den erfindungsgemäßen Materialien reichen -40 °C aus, um Ausbeuten über 50 % zu haben. Bei der verwendeten Mühle handelt es sich um eine Hosokawa Alpine Contraplex-Stiftmühle 160 C.

Alle Pulver wurden bei 100 µm gesiebt, um sicherzustellen, dass zu grobe Partikel den Bauprozeß nicht stören können. Alle Pulver wurden mit 0,1 Teilen Aerosil 200 ausgerüstet.

**Tabelle 2**

| | **D10** | **D50** | **D90** | **Schüttdichte** |
|---|---|---|---|---|
| | µm | µm | µm | g/l |
| Vergleichsbeispiel 1: EOSINT PPA 2200 | 39 | 55 | 79 | 430 |
| Vergleichsbeispiel 2: PEBA 1 | 30 | 70 | 126 | 361 |
| Vergleichsbeispiel 3: PEBA 2 | 57 | 126 | 208 | |
| Vergleichsbeispiel 4: PEBA 3 | 29 | 71 | 125 | 322 |
| Beispiel 1 | 33 | 74 | 127 | 364 |
| Beispiel 2 | 29 | 68 | 122 | 434 |
| Beispiel 3 | 30 | 71 | 126 | 358 |
| Beispiel 4 | 33 | 76 | 131 | 309 |
| Beispiel 5 | 29 | 69 | 127 | 324 |
| Beispiel 6 | 32 | 72 | 124 | 362 |
| Beispiel 7 | 29 | 68 | 124 | 354 |
| Beispiel 8 | 26 | 64 | 121 | 344 |
| Beispiel 9 | 27 | 70 | 120 | 333 |
| Beispiel 10 | 31 | 65 | 128 | 349 |

### Verarbeitung:

Alle Pulver wurden auf einer EOSINT P360 der Firma EOS GmbH, Krailling, verbaut. Es handelt sich um eine Lasersintermaschine. Der Bauraum wurde bis nahe an den Schmelzpunkt der jeweiligen Probe vorgeheizt. Die Parameter für den Laser, wie Geschwindigkeit, Leistung, wurden durch Ausprobieren jeweils an das Material angepasst. Die nicht erfindungsgemäßen Materialien waren deutlich schwieriger zu verarbeiten, insbesondere, was eine riefenfreie Auftragung der jeweiligen Pulverschicht betrifft.

Wie aus nachfolgender Tabelle zu ersehen ist, zeigen die erfindungsgemäßen Probekörper deutliche Vorteile besonders bei der Kerbschlagzähigkeit bei -30 °C, vorrausgesetzt, die Dichte der Bauteile kann auf einen Wert über 0,9 g/mm³ eingestellt werden. Vergleichen wir Vergleichsbeispiel 1 mit den Beispielen 9 und 10, so sehen wir immerhin eine Verdoppelung der Kerbschlagzähigkeit, und auch die anderen mechanischen Werte sind verbessert; die Teile sind allerdings weicher als Teile aus dem Referenzmaterial aus Beispiel 1. Betrachtet man die Vergleichsbeispiele 2-4 und die Beispiele 1-8, so stellt man deutliche Verbesserungen insbesondere der Kerbschlagzähigkeiten bei -30 °C fest. Die Bauteile aus Vergleichsbeispiel 2 sind so porös, dass die Bauteile so nicht einsetzbar sind.

**Tabelle 3**

| | **Elastizitäts modul** | **Zugfestig keit** | **Reiß dehnung** | **Kerbschlag RT** | **Kerbschlag -30** | **Dichte** |
|---|---|---|---|---|---|---|
| | N/mm² | N/mm² | % | kJ/m² | kJ/m² | g/mm³ |
| Vergleichsbeispiel 1: EOSINT PPA 2200 | 1700 | 48 | 15 | 4,7 | 3,1 | 0,95 |
| Vergleichsbeispiel 2: PEBA 1 | keine Prüfkörper | | | 31,7 | 64,9 | 0,8 |
| Vergleichsbeispiel 3: PEBA 2 | 101 | 12,5 | 277 | 42,8 | 14,6 | 0,92 |
| Vergleichsbeispiel 4: PEBA 3 | 220 | 15,4 | 25 | 7,3 | 4,0 | 0,82 |
| Beispiel 1 | 138 | 13,8 | 64 | 29,3 | 23,1 | 0,9 |
| Beispiel 2 | 173 | 11,7 | 38 | 43,7 | 1,8 | 0,82 |
| Beispiel 3 | 165 | 13,8 | 38 | 21,2 | 6,5 | 0,85 |
| Beispiel 4 | 77 | 6,6 | 48 | 22,4 | Kein Bruch | 0,94 |
| Beispiel 5 | 74 | 6,1 | 36 | 25,7 | Kein Bruch | 0,92 |
| Beispiel 6 | 90 | 6,6 | 30 | 30,5 | 42,3 | 0,97 |
| Beispiel 7 | 91 | 7,6 | 52 | Kein Bruch | Kein Bruch | 0,82 |
| Beispiel 8 | 81 | 7 | 46 | Kein Bruch | Kein Bruch | 0,82 |
| Beispiel 9 | 1008 | 28.1 | 18 | 12,8 | 9,9 | 0,92 |
| Beispiel 10 | 1222 | 32 | 16 | 9,2 | 7,8 | 0,93 |

## Patentansprüche

1. Polymerpulver zur Verwendung in einem schichtweise arbeitenden Verfahren, bei dem selektiv Bereiche der jeweiligen Pulverschicht durch den Eintrag elektromagnetischer Energie aufgeschmolzen werden,
**dadurch gekennzeichnet,**
**dass** das Pulver zumindest ein Blockpolyetheramid aus Oligoamiddicarbonsäuren mit einem mittleren Molekulargewicht (Gewichtsmitel) zwischen 1000 und 20000 g/mol und und Polyetheraminen mit einem mittleren Molekulargewicht (Gewichtsmittel) zwischen 200 und 5000 g/mol, aufweist, wobei das Blockpolyetheramidpulver eine Schmelztemperatur Tm von 140 bis 200 °C aufweist.

2. Polymerpulver nach Anspruch 1,
**dadurch gekennzeichnet,**
**dass** das Pulver zumindest Polyetherdiamine als Polyetheramine, aufweist.

3. Polymerpulver nach einem der Ansprüche 1 oder 2,
**dadurch gekennzeichnet,**
**dass** das Pulver zumindest Polyetheramine aufweist, wobei die Ethereinheiten alkylverzweigt sind.

4. Polymerpulver nach einem der Ansprüche 1 bis 3,
**dadurch gekennzeichnet,**
**dass** das Pulver zumindest ein Blockpolyetheramid aufweist, das hergestellt ist aus Oligoamiddicarbonsäuren mit einem mittleren Molekulargewicht (Gewichtsmittel) unterhalb 1500 g/mol.

5. Polymerpulver nach einem der Ansprüche 1 bis 4,
**dadurch gekennzeichnet,**
**dass** die Selektivität durch die Auftragung von Suszeptoren, oder Inhibitoren, oder Absorbern, oder durch Masken erzielt wird.

6. Polymerpulver nach einem der Ansprüche 1 bis 4,
**dadurch gekennzeichnet,**
**dass** die Selektivität durch die Fokussierung eines Laserstrahls erzielt wird.

7. Polymerpulver nach zumindest einem der Ansprüche 1 bis 6,
**dadurch gekennzeichnet,**
**dass** das Blockpolyetheramid durch Polykondensation erhalten wurde.

8. Polymerpulver nach zumindest einem der Ansprüche 1 bis 7,
**dadurch gekennzeichnet,**
**dass** das Blockpolyetheramidpulver durch Vermahlung erhalten wurde.

9. Polymerpulver nach mindestens einem der Ansprüche 1 bis 8,
**dadurch gekennzeichnet,**
**dass** das Blockpolyetheramidpulver nach der Vermahlung durch anschließende Klassierung erhalten wurde.

10. Polymerpulver nach mindestens einem der Ansprüche 1 bis 9,
**dadurch gekennzeichnet,**
**dass** die Anzahl von Aminoendgruppen zu Carboxylendgruppen um nicht mehr als 10 % voneinander abweicht.

11. Polymerpulver nach mindestens einem der Ansprüche 1 bis 10,
**dadurch gekennzeichnet,**
**dass** es einen Überschuss an Aminoendgruppen aufweist.

12. Polymerpulver nach mindestens einem der Ansprüche 1 bis 11,
**dadurch gekennzeichnet,**
**dass** es einen Katalysator aufweist.

13. Polymerpulver nach mindestens einem der Ansprüche 1 bis 12,
**dadurch gekennzeichnet,**
**dass** es Phosphorsäure aufweist.

14. Polymerpulver nach mindestens einem der Ansprüche 1 bis 13,
**dadurch gekennzeichnet,**
**dass** das Blockpolyetheramidpulver eine Lösungsviskosität zwischen 1,4 bis 2,1 aufweist (die Messung ist in der Beschreibung offenbart).

15. Polymerpulver nach mindestens einem der Ansprüche 1 bis 14,
**dadurch gekennzeichnet,**
**dass** das Blockpolyetheramidpulver eine Lösungsviskosität zwischen 1,5 bis 1,9 aufweist (die Messung ist in der Beschreibung offenbart).

16. Polymerpulver nach mindestens einem der Ansprüche 1 bis 15,
**dadurch gekennzeichnet,**
**dass** das Blockpolyetheramidpulver eine Lösungsviskosität zwischen 1,6 bis 1,8 aufweist (die Messung ist in der Beschreibung offenbart).

17. Polymerpulver nach mindestens einem der vorhergehenden Ansprüche,
**dadurch gekennzeichnet,**
**dass** das Blockpolyetheramidpulver einen mittleren Korndurchmesser zwischen 40 und 120 µm aufweist (die Messung ist in der Beschreibung offenbart).

18. Polymerpulver nach mindestens einem der Ansprüche 1 bis 17,
**dadurch gekennzeichnet,**
**dass** das Blockpolyetheramidpulver eine BET-Oberfläche kleiner als 5 m²/g aufweist (die Messung ist in der Beschreibung definiert).

19. Polymerpulver nach mindestens einem der Ansprüche 1 bis 18,
**dadurch gekennzeichnet,**
**dass** das Blockpolyetheramidpulver eine Rekristallisationstemperatur zwischen 50 °C und 190 °C aufweist (die Messung ist in der Beschreibung definiert).

20. Polymerpulver nach zumindest einem der Ansprüche 1 bis 18,
**dadurch gekennzeichnet,**
**dass** es Hilfsstoffe und/oder Füllstoffe aufweist.

21. Polymerpulver nach Anspruch 19,
**dadurch gekennzeichnet,**
**dass** es als Hilfsstoff Rieselhilfsmittel aufweist.

22. Polymerpulver nach Anspruch 19,
**dadurch gekennzeichnet,**
**dass** es als Füllstoff Glaspartikel aufweist.

23. Polymerpulver nach zumindest einem der Ansprüche 1 bis 22,
**dadurch gekennzeichnet,**
**dass** es organische und/oder anorganische Pigmente aufweist.

24. Polymerpulver nach Anspruch 23,
**dadurch gekennzeichnet,**
**dass** es Ruß aufweist.

25. Polymerpulver nach Anspruch 23,
**dadurch gekennzeichnet,**
**dass** es Titandioxid aufweist.

## Claims

1. Polymer powder for use in a layer-by-layer process in which regions of the respective powder layer are selectively melted via introduction of electromagnetic energy,
**characterized**
**in that** the powder comprises at least one block polyetheramide composed of oligoamide dicarboxylic acids with an average molar mass (weight-average) of from 1000 to 20 000 g/mol, and of polyetheramines with an average molar mass (weight-average) of from 200 to 5000 g/mol where the block polyetheramide powder has a melting point Tm of from 140 to 200°C

2. Polymer powder according to Claim 1,
**characterized**
**in that** the powder comprises at least polyetherdiamines as polyetheramines.

3. Polymer powder according to either of Claims 1 and 2,
**characterized**
**in that** the powder comprises at least polyetheramines, where the ether units have alkyl branching.

4. Polymer powder according to any of Claims 1 to 3,
**characterized**
**in that** the powder comprises at least one block polyetheramide prepared from oligoamide dicarboxylic acids with an average molar mass (weight-average) below 1500 g/mol.

5. Polymer powder according to any of Claims 1 to 4,
**characterized**
**in that** the selectivity is achieved via application of susceptors, or of inhibitors, or of absorbers, or via masks.

6. Polymer powder according to any of Claims 1 to 4,
**characterized**
**in that** the selectivity is achieved via focusing of a laser beam.

7. Polymer powder according to at least one of Claims 1 to 6,
**characterized**
**in that** the block polyetheramide has been obtained via polycondensation.

8. Polymer powder according to at least one of Claims 1 to 7,
**characterized**
**in that** the block polyetheramide powder has been obtained via grinding.

9. Polymer powder according to at least one of Claims 1 to 8,
**characterized**
**in that** the block polyetheramide powder has been obtained via classification subsequent to the grinding process.

10. Polymer powder according to at least one of Claims 1 to 9,
**characterized**
**in that** the number of amino end groups does not differ from that of carboxy end groups by more than 10%.

11. Polymer powder according to at least one of Claims 1 to 10,
**characterized**
**in that** it has an excess of amino end groups.

12. Polymer powder according to at least one of Claims 1 to 11,
**characterized**
**in that** it comprises a catalyst.

13. Polymer powder according to at least one of Claims 1 to 12,
**characterized**
**in that** it comprises phosphoric acid.

14. Polymer powder according to at least one of Claims 1 to 13,
**characterized**
**in that** the block polyetheramide powder has a solution viscosity of from 1.4 to 2.1 (measurement information being disclosed in the description).

15. Polymer powder according to at least one of Claims 1 to 14,
**characterized**
**in that** the block polyetheramide powder has a solution viscosity of from 1.5 to 1.9 (measurement information being disclosed in the description).

16. Polymer powder according to at least one of Claims 1 to 15,
**characterized**
**in that** the block polyetheramide powder has a solution viscosity of from 1.6 to 1.8 (measurement information being disclosed in the description).

17. Polymer powder according to at least one of the preceding claims,
**characterized**
**in that** the block polyetheramide powder has an average grain diameter of from 40 to 120 µm (measurement information being disclosed in the description).

18. Polymer powder according to at least one of Claims 1 to 17,
**characterized**
**in that** the block polyetheramide powder has a BET surface area smaller than 5 m²/g (measurement information being disclosed in the description).

19. Polymer powder according to at least one of Claims 1 to 18,
**characterized**
**in that** the block polyetheramide powder has a recrystallization temperature of from 50°C to 190°C (measurement information being disclosed in the description).

20. Polymer powder according to at least one of Claims 1 to 19,
**characterized**
**in that** it comprises auxiliaries and/or fillers.

21. Polymer powder according to Claim 19,
**characterized**
**in that** it comprises powder-flow aids as auxiliary.

22. Polymer powder according to Claim 19,
**characterized**
**in that** it comprises glass particles as filler.

23. Polymer powder according to at least one of Claims 1 to 22,
**characterized**
**in that** it comprises organic and/or inorganic pigments.

24. Polymer powder according to Claim 23,
**characterized**
**in that** it comprises carbon black.

25. Polymer powder according to Claim 23,
**characterized**
**in that** it comprises titanium dioxide.

## Revendications

1. Poudre polymère destinée à une utilisation dans un procédé fonctionnant en couches, selon lequel des zones de la couche de poudre respective sont fondues sélectivement par l'apport d'énergie électromagnétique, **caractérisée en ce que**
la poudre comprend au moins un polyéther-amide séquencé constitué d'acides oligoamide-dicarboxyliques ayant un poids moléculaire moyen (moyenne en poids) compris entre 1 000 et 20 000 g/mol et de polyéther-amines ayant un poids moléculaire moyen (moyenne en poids) compris entre 200 et 5 000 g/mol, la poudre de polyéther-amide séquencé présentant une température de fusion Tm de 140 à 200 °C.

2. Poudre polymère selon la revendication 1, **caractérisée en ce que** la poudre comprend au moins des polyéther-diamines en tant que polyéther-amines.

3. Poudre polymère selon l'une quelconque des revendications 1 ou 2, **caractérisée en ce que** la poudre comprend au moins des polyéther-amines, les unités éther étant ramifiées par des groupes alkyle.

4. Poudre polymère selon l'une quelconque des revendications 1 à 3, **caractérisée en ce que** la poudre comprend au moins un polyéther-amide séquencé, qui est fabriqué à partir d'acides oligoamide-dicarboxyliques ayant un poids moléculaire moyen (moyenne en poids) inférieur à 1 500 g/mol.

5. Poudre polymère selon l'une quelconque des revendications 1 à 4, **caractérisée en ce que** la sélectivité est obtenue par application de suscepteurs ou d'inhibiteurs ou d'absorbeurs ou par masquage.

6. Poudre polymère selon l'une quelconque des revendications 1 à 4, **caractérisée en ce que** la sélectivité est obtenue par la focalisation d'un faisceau laser.

7. Poudre polymère selon au moins l'une quelconque des revendications 1 à 6, **caractérisée en ce que** le polyéther-amide séquencé a été obtenu par polycondensation.

8. Poudre polymère selon au moins l'une quelconque des revendications 1 à 7, **caractérisée en ce que** la poudre de polyéther-amide séquencé a été obtenue par broyage.

9. Poudre polymère selon au moins l'une quelconque des revendications 1 à 8, **caractérisée en ce que** la poudre de polyéther-amide séquencé a été obtenue par classification ultérieure après le broyage.

10. Poudre polymère selon au moins l'une quelconque des revendications 1 à 9, **caractérisée en ce que** les nombres de groupes terminaux amino et de groupes terminaux carboxyle ne diffèrent pas de plus de 10 % l'un de l'autre.

11. Poudre polymère selon au moins l'une quelconque des revendications 1 à 10, **caractérisée en ce qu'**elle présente un excès de groupes terminaux amino.

12. Poudre polymère selon au moins l'une quelconque des revendications 1 à 11, **caractérisée en ce qu'**elle comprend un catalyseur.

13. Poudre polymère selon au moins l'une quelconque des revendications 1 à 12, **caractérisée en ce qu'**elle comprend de l'acide phosphorique.

14. Poudre polymère selon au moins l'une quelconque des revendications 1 à 13, **caractérisée en ce que** la poudre de polyéther-amide séquencé présente une viscosité en solution comprise entre 1,4 et 2,1 (la mesure est décrite dans la description).

15. Poudre polymère selon au moins l'une quelconque des revendications 1 à 14, **caractérisée en ce que** la poudre de polyéther-amide séquencé présente une viscosité en solution comprise entre 1,5 et 1,9 (la mesure est décrite dans la description).

16. Poudre polymère selon au moins l'une quelconque des revendications 1 à 15, **caractérisée en ce que** la poudre de polyéther-amide séquencé présente une viscosité en solution comprise entre 1,6 et 1,8 (la mesure est décrite dans la description).

17. Poudre polymère selon au moins l'une quelconque des revendications précédentes, **caractérisée en ce que** la poudre de polyéther-amide séquencé présente un diamètre de particule moyen compris entre 40 et 120 µm (la mesure est décrite dans la description).

18. Poudre polymère selon au moins l'une quelconque des revendications 1 à 17, **caractérisée en ce que** la poudre de polyéther-amide séquencé présente une surface BET inférieure à 5 m²/g (la mesure est décrite dans la description).

19. Poudre polymère selon au moins l'une quelconque des revendications 1 à 18, **caractérisée en ce que** la poudre de polyéther-amide séquencé présente une température de recristallisation comprise entre 50 °C et 190 °C (la mesure est décrite dans la description).

20. Poudre polymère selon au moins l'une quelconque des revendications 1 à 18, **caractérisée en ce qu'**elle comprend des adjuvants et/ou des charges.

21. Poudre polymère selon la revendication 19, **caractérisée en ce qu'**elle comprend des adjuvants de ruissellement en tant qu'adjuvants.

22. Poudre polymère selon la revendication 19, **caractérisée en ce qu'**elle comprend des particules de verre en tant que charges.

23. Poudre polymère selon au moins l'une quelconque des revendications 1 à 22, **caractérisée en ce qu'**elle comprend des pigments organiques et/ou inorganiques.

24. Poudre polymère selon la revendication 23, **caractérisée en ce qu'**elle comprend du noir de carbone.

25. Poudre polymère selon la revendication 23, **caractérisée en ce qu'**elle comprend du dioxyde de titane.
